# EUROPEAN PATENT SPECIFICATION

(11) **EP 3 725 254 B1**
(45) Date of publication and mention of the grant of the patent: **02.09.2026**
(21) Application number: 20000134.5
(22) Date of filing: 10.09.2013
(51) Int. Cl.: A61B 34/30, A61B 34/20, G06F 3/01, G02B 27/01, A61B 1/00

(54) **MICROSURGERY SYSTEM CONTROLLED BY A HEAD-MOUNTED DISPLAY**
MIKROCHIRURGISCHES SYSTEM MIT STEUERUNG DURCH EINE AM KOPF GETRAGENE ANZEIGEVORRICHTUNG
SYSTÈME DE MICROCHIRURGIE DIRIGABLE PAR UN AFFICHAGE MONTÉ À LA TÊTE

(30) Priority: 10.09.2012 IL 22186312
(43) Date of publication of application: 21.10.2020
(62) Document number(s) of the earlier application(s) in accordance with Art. 76 EPC: 13785660.5 / 2 903 551
(73) Proprietor: Elbit Systems Ltd., 31053 Haifa (IL)
(72) Inventor: SCHNEIDER, Ron, 3440503 Haifa (IL)
(74) Representative: Schuhmann, Albrecht

(56) References cited:
- WO-A1-02/100284
- WO-A1-2011/142165
- WO-A2-2011/053921
- WO-A2-99/65381
- US-A- 5 876 325
- US-A1- 2009 245 600

## Description

### BACKGROUND

### 1. TECHNICAL FIELD

The present invention relates to the field of medical imaging, and more particularly, digital surgical capturing and display. The claimed invention is defined in the appended claims.

### 2. DISCUSSION OF RELATED ART

Currently available techniques for microscope surgical procedures utilize a classic optical microscope. The basic classical surgical microscope is constructed of high quality optical components, a zoom objective lens, eyepiece for user view and an XY motor. The user (or surgeon) sees the relevant object through direct optical channels. There are techniques to fold the light rays in the microscope to design a more comfortable and ergonomic structure for the user. However, because the microscopes are based on direct light rays channels, they are limited in their ability and flexibility to locate and shift the eyepiece. In addition, due to the classic direct view method of the microscope, with no digital means and sensors, the users do not benefit the digital domain advantages.

During prolonged use of the microscope (in particular, surgical operations), the user must position his head fixed to the microscope for a long period of time. The long time that the user holds his head in the microscope may cause neck pains.

Currently available techniques of endoscopic procedures (laparoscopy, colonoscopy and the like) include projecting the video on large monitor/s fixed in the operating room (OR). The endoscope is coupled to a digital camera. The camera video is projected on a large display. The entire staff, in particular the lead surgeon, must view the operation when their head is fixed to the monitor direction. The lack of ability to move their heads, view each other and their instruments causes the surgery to be more complicated, risky and increases the operation time (in some of the cases it may also cause an increase in the number of staff members). It is highly difficult for the surgeon supporting staff to assist since they do not see his direct actions and do not share his perspective. US 5 876 325 A, WO 02/100284 and WO 2011/142165 disclose endoscopic systems including HMD interfaces.

US 2009/245600 A1 discloses an endoscopic system with gaze detection means.

WO 99/65381 A2 discloses an apparatus and method for tracking the direction of a user's gaze for use with optical instruments which form a viewable image of an object, such as microscopes, cameras, telescopes etc.

WO 2011/053921 discloses systems and methods of acquiring and displaying information during surgical procedures which includes one or more head mounted displays. The system is further disclosed to a have visual tracking and annotation system which uses an eye tracking system as input means.

### BRIEF SUMMARY

The invention is defined in claim 1. Further embodiments are defined in the dependent claims.

Consonant with some embodiments of the present invention, the claimed systems may be provided with elements such as: The processor and video sources can support multiple users having their own set of gaze direction tracker and display. The video can also be forwarded to different locations for consultation and teaching purposes; The video can be recorded for future analysis and presentation; The image of the main video source may be space-stabilized, allowing the positioning of other virtual instruments at directions convenient to the user, giving patients history, current vital signs, past test results, CT scans, and the like. The head tracker may be used to control steerable instruments in the operating room.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout.

In the accompanying drawings:

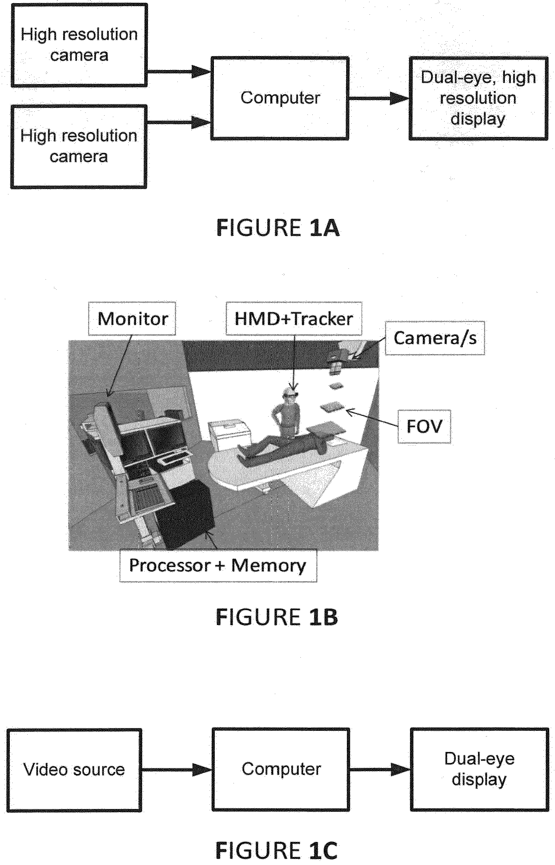

Figure 1A is a schematic block diagram illustrating one aspect according to some embodiments of the present invention;
Figure 1B is a perspective view illustrating another aspect according to some embodiments of the present invention;
Figure 1C is a schematic block diagram illustrating another aspect according to some embodiments of the present invention;
Figure 1D is a perspective view illustrating another aspect according to some embodiments not forming part of the present invention;
Figure 2A is a schematic block diagram illustrating yet another aspect according to some embodiments of the present invention;
Figure 2B is a perspective view illustrating yet another aspect according to some embodiments of the present invention;
Figure 2C is an optical diagram of yet another aspect according to some embodiments of the present invention;
Figure 2D shows graphs illustrating yet another aspect according to some embodiments of the present invention;
Figure 2E shows perspective views illustrating yet another aspect according to some embodiments of the present invention;
Figure 2F shows images illustrating yet another aspect according to some embodiments of the present invention; and
Figure 2G shows images illustrating yet another aspect according to some embodiments of the present invention.

### DETAILED DESCRIPTION

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

The present disclosure, in embodiments thereof, includes several main building blocks as follows:
high resolution camera or cameras having a large FOV; a head mounted display capable of projecting the desired resolution and FOV to the user; a head tracker, and an eye tracker. Embodiments of the present invention provide an angular resolution that is comparable or better than that of the human eye, and capable of object magnification similar to that of surgical microscopes (magnification up to ~30-40).

Additionally, some other elements may also be used as follow: a light source to illuminate the scene and object, processors, and interface devices. The system interfaces the users, operating-room equipment, and necessary infrastructure, such as communication.

According to some embodiments of the present invention, two systems for digital microscopy are provided herein. A third system, mainly for endoscope systems, is also described herein, but does not form part of the claimed invention. Many of the elements of the systems are common to both systems, and are described below, followed by a detailed description of the differences between the systems.

The proposed systems may use part or all of the following common elements. One such element is a video camera. The proposed systems use cameras to capture live images of the operated area. The image is displayed to the head mounted display (HMD) or monitor. The camera or cameras can be: Replacement to the microscope - the camera has a lens capable of focusing on objects at a distance of tens of centimeters. The camera or cameras can be add-on to the microscope - the add-on can be by using mechanical adapter to fit it in a dedicated port (surgical microscopes utilize beam splitter to split the light to the user's eyepiece and to additional ports to be used by cameras, other eyepieces and the like).

The user's gaze direction is measured by head and/or eye trackers. The camera captured images and gaze direction are routed to the system's processor. The processor crops relevant ROI from the high resolution camera images. The ROI images resolution are fit to the display resolution. The ROI location (or coordinates) is calculated according to the gaze direction.

The processor has input data ports for important medical data. The data can be patient history data, monitoring data or any external medical device.

The processor performs image processing on the captured images. The processing can be: Image correction, like fixed pattern noise corrections, color corrections, distortion corrections, histogram enhancement and the like; Adding markers on relevant image fixtures, tracking objects, emphasizing objects and the like; Overlaying monitoring data or patient history data; Fusing the video with other imaging device like CT, MRI etc.

The processor transmits the captured ROI images into display ports. The display device can be one of the following: High quality and high resolution color HMD. The HMD can be dual- eye display for stereo video or single-eye display for mono video. A part from the HMD the display can be any monitor or projecting device displaying the image. The monitor can be 3D monitor or standard 2D monitor. All the data or part of it can be saved on the system memory. The control on the system activation and important functions can be done using foot pedal, key-pad control box or voice activation.

Several types of systems are described herein. The differences between the systems are detailed below.

System I is a system for digital surgical microscopy. In this system the camera or cameras capture wide FOV area, larger than the display FOV. The camera resolution is very high so it is capable of capturing the entire FOV with all the necessary details in it (for large magnification purpose). After first adjustments, the system is of no need with mechanical motions mechanisms, like XY movement or optical zoom. Because the camera captures the entire FOV of interest, data magnification or areas of interest are extracted digitally.

System II is a system for digital surgical microscopy. The camera or cameras capture wide field of view (FOV) area, larger than the display FOV. The camera resolution is very high but may need mechanical motions, like XY movement or optical zoom to capture extended FOV.

System III is a system for digital surgical endoscopy, and thus does not form part of the claimed invention. In this type of system video is received from external imaging source like laparoscopy, colonoscopy, neurosurgery, MRI, CT etc.

Consonant with embodiments of the present invention, the camera used in the systems maintains the standard required from high end surgical microscopes which support the human high optical performance standards. That is, sharp 3D image while maintaining situational awareness for the FOV provided by the microscope. The camera maintains these benefits and provides the user with increased functionality and situational awareness, while detaching him from the microscope. The camera main features are: 3D camera with ultra high resolution; 3D camera with resolution to cover the full FOV of the microscope; and Camera with ROI slaved to the HMD.

In one case the camera can be added on to surgical microscopes. Using adaptors and optics the camera will be connected to the microscopes. In another case, the camera will be with an objective lens (or photographic lens) which will be used to replace the entire surgical microscope. The camera will image the object of interest and transmit the image to the processor unit and to the display (monitor or HMD). It is crucial to maintain small time latency from the grabbing of the image to the image display. The small latency is crucial so the user will not suffer from delay between movements in the object plane till the image is displayed.

In some embodiments, two cameras are used to capture the images of the left and right eye (provided by the microscope) for stereo imaging. The cameras are positioned in two different angles to provide the display with images from different angles that will eventually create 3D imaging. This configuration may be implemented in parallel to the existing eyepieces or by replacing them (by either upgrading existing microscopes or replacing them with new instruments).

In other embodiments, one or more cameras can be used to capture images from different positions. The capturing can be done for fix location of the camera/cameras or by changing the cameras position to different locations. At every location the cameras captures the image and the processor saves the images in the memory. The images captured from the camera/cameras can be used to generate 3D model of the object. It can be made at real-time for fixed cameras or non-real-time for moving cameras. Example use for this embodiment can be creation of 3D model of the head and brain for neurosurgery. The camera/cameras are moved to different locations around the subject head. The images are saved in the memory. The processor uses the images to generate 3D model of the head/brain. Example algorithm for such 3D model can be implemented using radon transform. This model can be used during the surgery to help the surgeon navigate or improve the 3D orientation. The 3D model can be displayed to surgeon in 3D display, head mounted display, 2D display as 3D model. The 3D database can also be used for non-display purpose. The processor can perform different algorithms on the database, like image correlation, tracking and the like.

According to some embodiments, where a plurality of displays and a plurality of users of several types are present, the computer processor may be configured to apply a plurality of predefined image processing or video processing algorithms to at the at least one selected ROI and present outputs of the algorithms over the plurality of displays. In this manner, each user may select his or her own ROI and receive a tailored processed image by applying the image processing algorithms or any other operation applied to the user-specific ROI and display. By the aforementioned embodiments, the computer processor becomes means for managing the systems resources in terms of image sensors, input devices, ROIs to be selected and the various displays present on the scene.

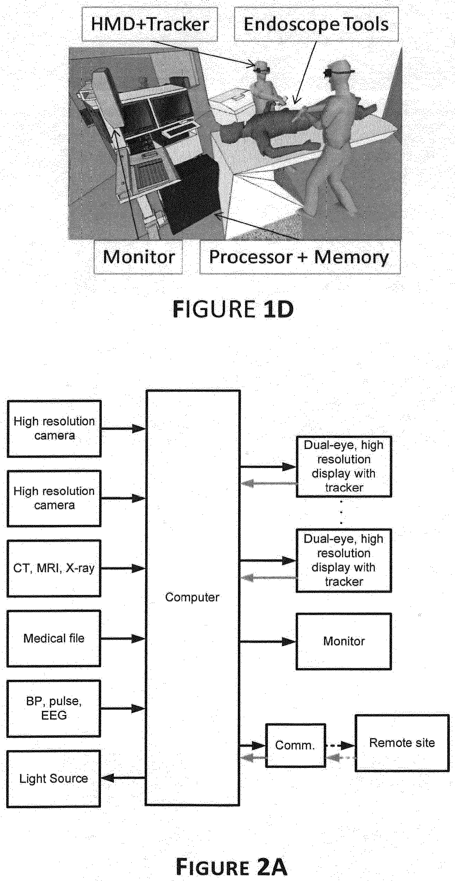

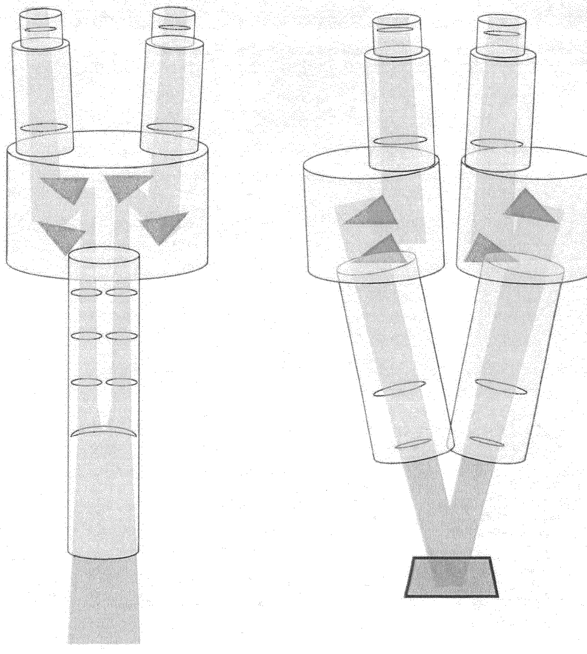

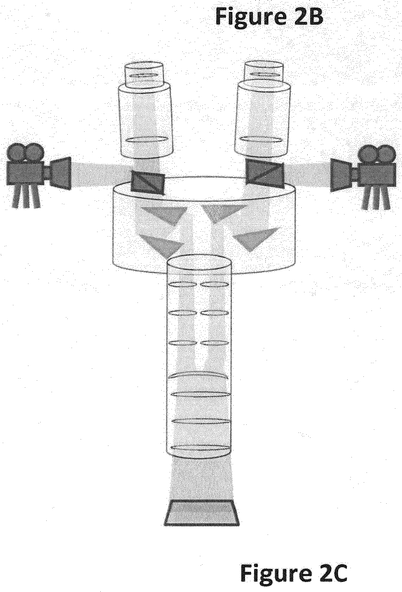

As shown in Figure 2B, some of the surgical microscopes employ a common main objective (CMO) design. A major advantage of this design is the ability to incorporate prisms into the optical path which allow the diversion of some of the light without impacting substantially the quality of the image. Cameras can be placed at some of the predesigned beam splitting location to capture the image of each of the optical paths, one for each eye, as shown in Figure 2C.

Another method of designing the surgical microscopes is by using the Greenough design, which gives several advantages. This design allows the location of the microscope at a large distance from the patient. The large distance is important for lowering the risk of patient contamination. The design of the microscope is simpler, makes the instrument production cheaper, and supports higher numerical apertures and greater depths of field. The use of digital cameras enables optical distortions correction, such as the keystone effect, and introduces accessories independent of the optical design, much like the CMO design. In the case where the cameras are add-on to microscopes with robotic (motorized) arm, the motor can be enslaved to the head and eye movements - a benefit in some fields of brain surgery.

Referring now to the surgical microscope camera, in the first system concept decried above (system I), the camera captures the full FOV of the microscope. The camera resolution is high enough so the system angular resolution is comparable to that of the human eye. The cameras can be assembled as stand-alone or attached to the microscopes using the CMO or Greenough designs. From the captured images, ROIs are cropped and sent to the display unit. The ROIs coordinates depend on the LOS of the head and/or the eye. The ROI coordinates can be also extracted from joystick, keyboard, foot pedestal, voice activation and any other input device. Capturing the full FOV at maximum resolution makes it feasible to display the user with a human-eye-like resolution for its area of interest. This working method allows several benefits to the user: providing the user with a superb image; zooming in to the image digitally; digital XY motion in the image; providing a different user with an image of its area of interest simultaneously to the primary user; recording the entire FOV of the microscope for debriefing and later analysis; and sharing the image observed by the user for the purpose of teaching or receiving assistance.

The cropping of the ROI out of the full frame can be carried out in several methods. The first method involves grabbing the full image out of the sensor. In this method the ROI is digitally cropped from the full image after the grabbing. The full image or only the ROI can be stored in the processor memory. The second method involves grabbing only the ROI from the sensor and not the full frame. The grabbing can be made by addressing only the relevant pixels in the image array. In the first two methods, the processor performs image resize on the ROI so it will fit the resolution of the display unit. The third method involves grabbing and under-sampling of the ROI from the sensor. In this method the digital image sensors are configured to under-sample for adjusting the spatial resolution of the video sequence to the spatial resolution of the electronic display. An example of under-sampling is the sampling of every second pixel in the array instead of every pixel.

In the first method, using an ROI digitally cropped from the full image, the ROI is a powerful tool for improving surgery. When the full high resolution is grabbed to the processor memory important data can be used for different applications. In the simplest way, the entire data can be saved for late debrief. During the operation, each user can select different ROI, according to required coordinates and magnification, without disturbing the other users. This is important, as an example, for complicated surgeries, were more than one surgeon are operating at the same time. Not only surgeons can use the different ROI's, but students, nurses or other staff members, users which are not in the same operating room etc. The ROI's can be used for multiple users, were the other user is the processor. For example, the surgeon can mark an area of interest, where the processor tracks that there is no blood flow or other defined event. The user can mark different ROI's with bookmarking. In this case the user can return to the bookmarked ROI in a frame time. The time transition from ROI to ROI is frame time. It includes magnifications change (from large to small values), XY change or emergency change.

The second system concept described above (system II) is to enslave the LOS of a narrow FOV camera, having the same angular resolution as the above camera, to the head and/or eye tracker. This allows the capturing of smaller resolution images, decreasing the amount of data to be handled by the system, while still maintaining the necessary resolution. A fast bi-axial steering mirror scans the image obtained through a large FOV optics onto a small FOV camera. The position of the mirror is enslaved to the user's head and eye movements, replacing the cropped area of the previous solution. Additional method for shift the LOS of the camera is by using pan and tilt mechanism of some elements of the system.

In another embodiment there is a combination of the first and second concepts. In this concept the camera has a moderate resolution with scanning possibilities. In addition there is internal ROI to display on the HMD or monitor.

The HMD may be in the form of a display device, worn on the head, which has a small display optic in front of one or each eye. The HMD can produce 2 dimensional images or 3 dimensional images (stereoscopic display). In the context of the present invention, the HMD is referred as a tool for displaying the video, symbols, or any type of displayable information. To achieve high quality video, the HMD can be implemented displaying color image, 3D (for every eye), high resolution and high FOV. However, one or more of the mentioned qualities might not be implemented.

A see-through HMD may be used in some embodiments, thus allowing a video to be superimposed on a real-world view. For example, superimposing real-world view with video can be done by projecting the video through a partially reflective surface and viewing the real world directly. Combining real-world view with video can also be done electronically by accepting video from a camera capturing the real world and mixing it electronically with video from the system.

Usually HMD images are focused to infinity. Focus to infinity provides the viewer relaxed effort for the eye and less exhausting. However, when using see-through HMD, and the real world objects are not located at infinity (for example, closed room), the focus of the HMD needs to be set to the objects distance. In general, HMDs can utilize a dynamic focus mechanism, setting the projected image focus distance to the viewer eye focus distance (or to the distance of the object that the viewer sees).

The HMD of the system of the claimed invention utilizes a head tracker. The head tracker is used to locate the user head location in space and by so calculating the viewer LOS (line of Sight). Knowledge of the line of sight of the viewer is a powerful tool allowing different unique functions and applications. For example, in specific, a see-through HMD with head tracker can be implemented to fix a virtual video in space (set fixed relative to the real world), to display data in any real world location. In a general manner, it can be used to fix any data or video to a specific coordinate in space.

Additionally, the user LOS may be used for different applications. For example, the LOS can be used as a controller for microscope motors (for translator motion or zoom) instead of a joystick. Another example is the change of an ROI (region of interest) in the displayed image. The change in ROI can be its coordinate in the image or its size (digital zoom).

The HMD utilizes the head tracker so it is possible to present symbols, information, images and the like in the field of regard of the user. The FOV can be defined around a specific line of sight (for example directly to the object under operation). The field of regard can be defined around the FOV.

According to some embodiments, the computer processor may be configured to project the video sequences over the electronic display in a first and a second regions, wherein an angular resolution of the second region is higher than a threshold such as 0.0005 radians and wherein the angular resolution of the first region is lower than the aforementioned threshold of 0.0005 radians, and wherein the first region has a field of view of at least of for example 2° and is substantially centered around the LOS of the user.

The HMD can utilize eye tracker. The eye tracker can be used to locate the exact eye LOS relative to the HMD device. Knowledge of the eye LOS can be used to implement smart displays were the projected image is depended on the observer eye LOS. In general, most of the functions mentioned regarding the head tracker can be implemented using the eye tracker. In combination with the head tracker, the HMD can be a very friendly tool to visualize data fixed in space and to be used as controller.

In order to meet the present invention, the HMD must produce high quality video. The HMD quality of video must be acceptable for the user, when compared to the image viewed through regular (not digital) microscope eyepiece. To achieve high quality video from the HMD several critical parameters must be implemented - high angular resolution, wide FOV, high color quality and high dynamic range. In the scope of this invention we refer only to the first two parameters - high angular resolution and wide FOV.

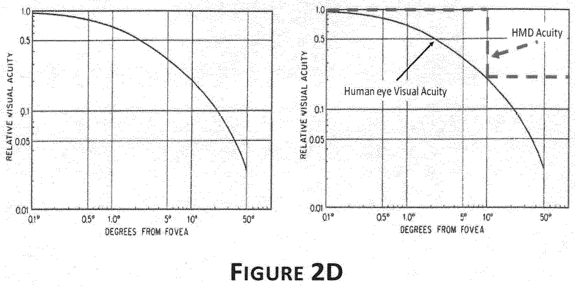

The user of the HMD is the human viewer. To achieve high quality display, the HMD optical parameters need to be good enough to satisfy the human vision. The FOV of the human vision is very large. Yet, the human angular resolution (or visual acuity) is depended on the location of the image on the retina. There is a small area (small FOV) of the retina with improved visual acuity. This area is called the fovea, and covers about 2 degrees of visual FOV. When drawing away from the fovea the visual acuity degrades monotonically (see Figure 2D, left image). The human viewer will not notice improvement in resolution or have the ability to see more details using HMD with smaller angular resolution than the human vision (see Figure 2D, right image). So, in general, a high quality HMD will have angular resolution similar or better than the human vision.

Referring to surgical microscope HMD, two exemplary and non-limiting implementations of HMD which will produce a high quality image, and which will have small enough angular resolution, so the human viewer will not feel the effect of observing digital image are described herein:
The first implementation of the HMD is by producing constant angular resolution as a function of the FOV. In general this approach utilizes a high resolution display as part of the HMD.

The second implementation of the HMD is by producing varying angular resolution as a function of the HMD FOV. The angular resolution must be better than the human vision, so in this implementation, small angular resolution must be projected in the FOV of the fovea line of sight. In the peripheral FOV (around the fovea) the angular resolution may be larger than in the fovea area (Figure 2D). The HMD has a mechanism to shift the projected image (to the viewer) according to eye motion (using eye tracker). The direction of the projected image in this case follows the viewer LOS, so it is depended to the eye motion; the viewer sees an image in front of him.

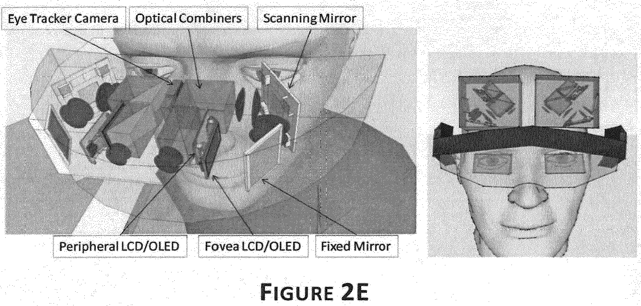

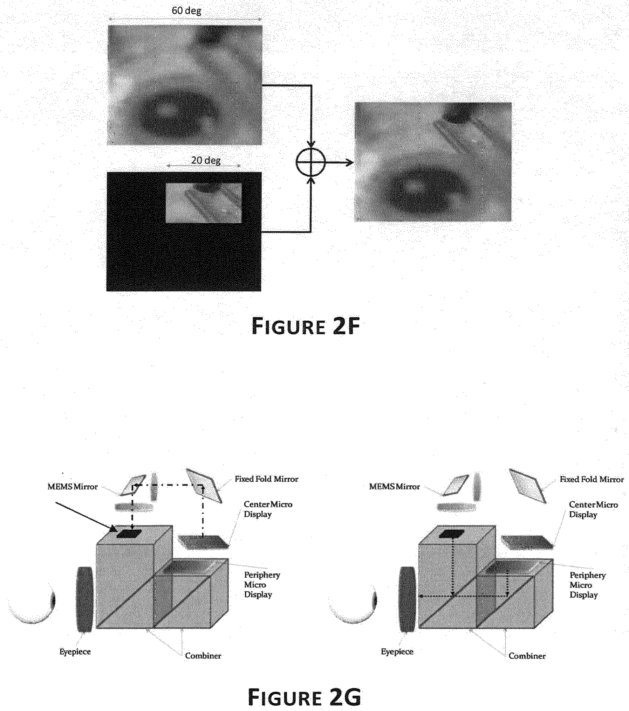

Example for a design of the second implementation of the HMD (producing varying angular resolution) is illustrated in Figure 2E. In the example there are two micro-displays for each eye. One micro-display is used for the peripheral image. The second micro-display is being used for the fovea image. The second micro-display is projected at a narrow FOV relative to the second micro-display. The two projected images (of the two micro displays) are combined using an optical combiner. With an eyepiece the viewer can see the combined image (Figure 2F). To achieve better transition between the two superimposed images some smoothing can be done in the stitching area. For example, in the edges of the fovea image, the resolution can be degraded monolithically (using image processing smearing) down to the resolution of the peripheral image. This will cause super-imposing the images to look more natural.

The image seen by the viewer must be with angular resolution better than the human visual acuity. To achieve that with the second implementation of the HMD, the fovea image micro-display (center micro-display) must be moved according to the LOS of the viewer. One way to achieve it is by using mirror (MEMS for example) to shift the center micro-display image on the eyepiece focal plane. The rotating mirror (2 Axis) can be shifted using an eye tracker - enslaving the mirror to the viewer LOS. An example implementation is shown in Figure 2G. The optical components described in Figure 2F can be assembled directly in front of the eye or as a see-through HMD.

The HMD projected image can be achieved using micro-display (LCD, OLED, LCOS), DMD (Digital Micro-mirror Device), DLP (Digital Light Processing), scanning mirrors or any other method. In general the HMD can be implemented as a see-through device where the viewer can see the displayed image and the scenery behind it. The HMD can be implemented also as a non-see-through device where the viewer can see only the image that conceals the scenery behind it. The HMD can be implemented as a wireless or wired device communicating and receiving images from an external source.

Referring now to the head and eye trackers, some of the uses of the trackers may include: controlling the movement of robotic arm; controlling the movement of the ROI in the high resolution display; controlling the image Zoom; stabilizing the image in space; controlling the see-through brightness. Head and eye trackers are powerful tools. Their main role in the system application is to provide the LOS of the user's area of interest. In general, the head tracker provides the LOS for the displayed image FOV, while the eye tracker provides the LOS for the high resolution FOV. The LOS provided by the head trackers allows to crop the relevant ROI from the ultra-high resolution image or to direct the fast steering mirror to the appropriate angle. This image is then decimated before being displayed by the peripheral display.

The head tracker LOS enables the HMD to create space-stabilized objects. These objects include the image from the microscope and any further data that needs to be available to the user, such as medical history, previously obtained CT scans, current patient vital statistics, as measured by other O.R. instruments, etc. These may be placed at user-defined spatial locations. For example, looking at 45° to the left, the user can see the patient's last CT scan, looking forward gives the microscope image, looking down allows to see the patient through the display, and looking up provides the time display. For many users, the ability to provide a stable image is crucial for the prevention of nausea.

As mentioned above, the head tracker may also be used to perform other tasks, via the user interface, or as a source for directing microscope movements or other medical equipment.

Reference is now made to a wide FOV situation awareness (SA) camera. The SA camera may not be part of the normal optical channel of the microscope. Optical microscopes do not provide the user the tools for SA. When the user eyes are fixed to the microscope eye piece the vision is limited to the limited FOV of the scope. Events that occur outside the microscope are hidden from the user. In addition there is no ability to maneuver the microscope LOS to different object based on seeing.

Embodiments of the present invention may provide further increase of the peripheral FOV with extended SA. The increase of FOV is provided using lower angular resolution camera or cameras. The additional cameras may be located outside the microscope, in a perimeter close to the microscope objectives. The wide FOV cameras images may be fused (or superimposed) with the images obtained through the microscope optics and provide further peripheral FOV. Another technique for displaying the wide FOV images is in a different window in the HMD field of regard, or to present the wide FOV images overlapping part of the microscope image.

Reference is now made to voice activation of the system. Controlling the system may be implemented in several ways, via dedicated control panels, specially designed levers and buttons to be used by the user using his/her feet, etc. Of special interest is the voice activation technology, which allows hands-free control, leaving the user's hands free to perform the additional tasks, while providing a flexible and a rich interface. Voice recognition allows the identification of pre-programmed key-words, reserved for specific purposes, to be used by any user, and to allow users to program their own key-words, independent of a language or dictionary. For example, the oral command "markers on" may add pre-defined markers onto the displayed image. A Spanish-speaking user may supplement the English commands with its native language commands by programming them himself.

Voice activation can be used in conjunction with the head and eye trackers to construct elaborate yet intuitive user interface. For example, the "zoom on" oral command may enable a head movement dependent image zoom. Moving the head forward zooms into the image, while moving it backwards zooms out. The "zoom off" command turns off this feature, allowing for normal operation.

Referring now to the main processor and memory unit, the main processor unit is used primarily to: interface with the camera, the HMD, the head tracker, the eye tracker, the video monitors, the voice activation hardware, any controller panels and the microscope; running different real time software applications; running video editing software applications; playing the video from the memory unit; interfacing other systems like MRI, CT and the like. The memory unit is used primarily to record the video or images from the cameras and running real time software applications.

Referring now to the main processor unit interfaces, the processor interface to the camera and includes camera gain control, exposure control and any other control setting the camera working point. In addition the processor sets the video frame rate and ROI to capture from the camera. The processor unit captures the video from the camera and if the user selects to record the data, pushes the video to the memory unit. The processor interface to the head tracker includes calculation of the LOS of the head. The head LOS can be calculated for 3 coordinates (azimuth, elevation and rotation) or six coordinates (also location in space) for more accurate applications. The processor interface to the eye tracker includes calculation of the LOS of the eye. The processor interface to the HMD includes HMD brightness control, contrast control and any other control setting the HMD displayed image. According to the eye and head tracker inputs, the processor pushes the HMD the relevant images, data and symbols. For complicated displays involving superposition of two micro displays (Figure 2F) the processor defines the images to push to the different micro displays. The processor may control or transfer relevant data for hardware in the HMD like the scan mirrors, head tracker, eye tracker camera or any other hardware.

The following are exemplary main processor unit real time applications. Head tracker applications may include: Controlling the microscope motors or ROI for translator movement and for optical or digital zoom. For example, when the user moves the head to different directions, microscope motors follows the head movement. In this way, for example, the viewer can divert the microscope to the desired object. The control of the optical zoom can be by moving the head toward and backward, setting the zoom in and zoom out function.

Another application is changing the brightness of the see-through HMD projected display. For example, the user can define that when looking in a specific direction the projected image is bright, and when moving the head to different location the image turns darker to enable higher appearance of the real world. According to the head location, the displayed image can be video, different symbols or different information.

Another embodiment is changing the see-through transparency as a function of the user head location. When the user's head is in one direction the transparency is low, so the projected image contrast is high. When the head moves to another direction, the transparency is high, so the user can have situation awareness through the HMD. Combination of the display brightness reduction can be used also, to improve the contrast of the background image. In general, the transparency change can be made on part of the see-through display or all of it. Transparency may be adjusted according to user manual selection and/or other ambient thresholds.

The transparency change can be made using passive or active coating/pads on the see-through display. These coating/pads are placed on a display element which is in the user line of sight. In general, it can be placed on visor, combiner, waveguide or any other optical element used for the see-trough display. The passive coating can decrease the see-through display transmission by using UV/NIR illumination on it. The active coating decrease transmission when electrically activated.

Eye tracker applications may include: For complicated displays involving superposition of two micro displays (Figure 2F) the eye tracker defines the location of the smaller angular resolution image (determines the image location on the eye piece focal plane). Shifting the entire projected display LOS.

According to the LOS of the viewer the see-through HMD can focus the projected image according to the real world object distance. With additional mechanism (like a camera) and the LOS of the viewer the HMD can detected the distance to the object and focus accordingly. This focus mechanism will prevent fatigues to the eye (due to changes in focus between the display and the real world). Applications that were described using the head tracker can be used also using eye tracker up to the extent of the human vision FOV.

The system and image processing applications may include: The processor generates different markers of interest features on the projected imaged. In the case of surgery the markers can be based to specify features to address. The processor generates important OR (operating room) equipment data and patient history information to present on the HMD projected display. The data can be presented on the video displayed or in the field of regard. Markers can be pushed from automatic data management or personal.

The above systems may utilize a light source. The most common approach is a light source designed to illuminate from the cameras optical line of sight. In this way the lens is built in such a way that the light from the source is directed through mirrors or beam splitters to illuminate through the lens. One more common approach is to illuminate from a singular point at some location near the lens. A third approach may be to use more than one light source. The multiple sources can be the ones described in the first two approaches. In addition a ring of light sources can be designed around the lens. The distribution of light sources produces uniform light with less shadowing in the image.

Once using the light sources it is possible to utilize the digital domain to control camera and the light source. For the camera it is common to have algorithms like AGC (automatic gain control) and AEC (automatic exposure control) to calculate and set the camera working point. The gain and exposure are usually calculated to produce image with maximum dynamic range and minimum saturations. With a single or multiple light sources an ALC (automatic light control) may be used. Based on the image the algorithm calculates the light levels of the single or multiple light sources. The controllable light sources facing the scenes are dynamically adjusted based on a dynamic analysis of the image histogram of the video sequences. The level of the lights are adjusted to optimize the contrasts, dynamic range and to minimize specular reflections from objects.

For some applications and procedures it is important to capture images in specific and limited spectral bands. For example capturing images in the NIR (near infra read) spectral band can enhance blood vessels. The light sources may be designed to radiate different spectral bands. One method to achieve the different spectrums is by using different light sources. For example the use of different types of LEDs, in different colors or in the NIR. A second method to achieve different spectrums is by using optical filters, like band pass filters in front of the light sources. One source can be covered by one type of filter, while a different source will be covered by a second type of filter.

Instead of designing the light sources for different spectrums, or in addition, the camera can be designed to capture images in different spectral bands. The camera optics may be designed so in some location in the optical path an optical filter is inserted. The mechanism of the optical filter can be designed to be fixed permanently or to support removal and insertion of the filter. The mechanism is not limited to one filter. For example a filter wheel or a light prism can be used to support multiple types of filters for different spectral bands. While performing the procedure, the user can set the spectral band by setting the corresponding optical filter.

The above systems may utilize a projector illuminating the imaged object. The projector can illuminate in the visible spectrum or in the near-infrared spectrum. The visual illumination can provide some visual guidance to the users. For example, to mark areas of interest, mark places to perform surgery cuts, direction symbols etc. Illuminating in the near-infrared can be used for structured light applications. The structured light is used to produce 3D data base of the objects. The projector can be of any type - DMD, Pico MEMS projector, LCD, CRT etc.

Another embodiment of the invention is guidance for the user. The guidance can be done by creating a path for the surgeon tools. The path is created using the 3D database created from images received from the cameras. Based on these images the system tracks the tool and guides the surgeon to the designated direction. The path can be generated automatically, manually or by a combination of both. The system can generate warning when the surgeon does not move the tool in right direction or gets close to sensitive areas.

In the above description, an embodiment is an example or implementation of the invention. The various appearances of "one embodiment", "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment. Embodiments of the invention may include features from different embodiments disclosed above, and embodiments may incorporate elements from other embodiments disclosed above. The disclosure of elements of the invention in the context of a specific embodiment is not to be taken as limiting their used in the specific embodiment alone.

Furthermore, it is to be understood that the invention can be implemented in various ways and that the invention can be implemented in embodiments other than the ones outlined in the description above.

The invention is not limited to those diagrams or to the corresponding descriptions. For example, flow need not move through each illustrated box or state, or in exactly the same order as illustrated and described.

Meanings of technical and scientific terms used herein are to be commonly understood as by one of ordinary skill in the art to which the invention belongs, unless otherwise defined.

While the invention has been described with respect to a limited number of embodiments, these should not be construed as limitations on the scope of the invention, but rather as exemplifications of some of the preferred embodiments. Other possible variations, modifications, and applications are also within the scope of the invention, as long as they fall within the scope of the appended claims.

## Claims

1. A digital surgical microscopy system comprising:
at least one camera, configured to acquire images of an operated-area of a patient;
a head mounted display (HMD), configured to display to a user at least a region of interest (ROI) in said images;
a tracker configured to track said user's head movements; and
a processing device configured to be coupled with said HMD, said at least one camera, and said tracker, said processing device configured to control at least one function of said digital surgical microscopy system according to said movements,
wherein said at least one function includes at least one of: changing an optical or digital zoom, changing a size of said ROI, changing a location of said ROI, and controlling microscope motors;
wherein said digital surgical microscopy system is a non-endoscopy system.

2. The digital surgical microscopy system according to claim 1, wherein said at least one function further includes changing an image displayed via said HMD.

3. The digital surgical microscopy system according to claim 2, wherein said image displayed via HMD includes either one of: said ROI, a patient medical history data, a patient pre-acquired scan, patient vital sign data, and operating room equipment data.

4. The digital surgical microscopy system according to claim 1, wherein said microscope motors include at least one of an optical zoom motor, and XY motor.

5. The digital surgical microscopy system according to claim 1, wherein said at least one function further includes at least one of controlling see-through transparency of said HMD, and changing the brightness of said HMD.

6. The digital surgical microscopy system according to claim 1, wherein said digital surgical microscopy system employs voice activation for enabling said control of said at least one function.

7. The digital surgical microscopy system according to claim 1, further including light sources configured for illuminating said operated area.

8. The digital surgical microscopy system according to claim 1, wherein said at least one camera is coupled with a robotic arm, wherein said at least one function further includes controlling movement of said robotic arm.

9. The digital surgical microscopy system according to claim 1, wherein said processing device is configured to perform image processing on said images acquired by said at least one camera.

10. The digital surgical microscopy system according to claim 9, wherein said image processing further include fixed pattern noise corrections, color corrections, distortion corrections, histogram enhancement, tracking objects, emphasizing objects, overlaying monitoring data, overlaying patient history data, and fusing with other image sources.

11. The digital surgical microscopy system according to claim 1, wherein said at least one camera includes two cameras configured for capturing a stereoscopic image.

12. The digital surgical microscopy system according to claim 1, further comprising a plurality of HMDs for each user, enabling each user to select their respective ROI.

## Patentansprüche

1. Ein digitales chirurgisches Mikroskopiesystem, umfassend:
mindestens eine Kamera, die dazu ausgelegt ist, Bilder eines Operationsbereichs eines Patienten aufzunehmen;
ein Head-Mounted-Display (HMD), das dazu ausgelegt ist, einem Benutzer mindestens einen Untersuchungsbereich (ROI) in den genannten Bildern anzuzeigen;
einen Tracker, der dazu ausgelegt ist, die Kopfbewegungen des Benutzers zu verfolgen; und
eine Verarbeitungsvorrichtung, die dazu ausgelegt ist, mit dem HMD, der mindestens einen Kamera und dem Tracker gekoppelt zu werden, wobei die Verarbeitungsvorrichtung dazu ausgelegt ist, mindestens eine Funktion des digitalen chirurgischen Mikroskopiesystems entsprechend den Bewegungen zu steuern,
wobei die mindestens eine Funktion mindestens eines der folgenden Elemente umfasst: Ändern eines optischen oder digitalen Zooms, Ändern einer Größe des ROI, Ändern einer Position des ROI und Steuern von Mikroskopmotoren;
wobei das digitale chirurgische Mikroskopiesystem ein nicht-endoskopisches System ist.

2. Das digitale chirurgische Mikroskopiesystem nach Anspruch 1, wobei die mindestens eine Funktion ferner das Ändern eines über das HMD angezeigten Bildes umfasst.

3. Das digitale chirurgische Mikroskopiesystem nach Anspruch 2, wobei das über das HMD angezeigte Bild eines der folgenden Elemente umfasst: den ROI, Daten zur Krankengeschichte des Patienten, einen zuvor erfassten Scan des Patienten, Daten zu den Vitalparametern des Patienten und Daten zur OP-Ausstattung.

4. Das digitale chirurgische Mikroskopiesystem nach Anspruch 1, wobei die Mikroskopmotoren mindestens einen optischen Zoom-Motor und/oder einen XY-Motor umfassen.

5. Das digitale chirurgische Mikroskopiesystem nach Anspruch 1, wobei die mindestens eine Funktion ferner mindestens eine der folgenden Funktionen umfasst: das Steuern der Durchsichtigkeit des HMD und/oder das Ändern der Helligkeit des HMD.

6. Digitales chirurgisches Mikroskopiesystem nach Anspruch 1, wobei das digitale chirurgische Mikroskopiesystem Sprachaktivierung nutzt, um die Steuerung der mindestens einen Funktion zu ermöglichen.

7. Digitales chirurgisches Mikroskopiesystem nach Anspruch 1, das ferner Lichtquellen umfasst, die zur Ausleuchtung des Operationsbereichs ausgelegt sind.

8. Das digitale chirurgische Mikroskopiesystem nach Anspruch 1, wobei die mindestens eine Kamera mit einem Roboterarm gekoppelt ist, wobei die mindestens eine Funktion ferner die Steuerung der Bewegung des Roboterarms umfasst.

9. Das digitale chirurgische Mikroskopiesystem nach Anspruch 1, wobei die Verarbeitungsvorrichtung so konfiguriert ist, dass sie eine Bildverarbeitung an den von der mindestens einen Kamera aufgenommenen Bildern durchführt.

10. Das digitale chirurgische Mikroskopiesystem nach Anspruch 9, wobei die Bildverarbeitung ferner die Korrektur von Rauschen mit festem Muster, Farbkorrekturen, Verzerrungskorrekturen, die Verbesserung des Histogramms, das Verfolgen von Objekten, das Hervorheben von Objekten, das Überlagern von Überwachungsdaten, das Überlagern von Patientendaten aus der Anamnese sowie die Fusion mit anderen Bildquellen umfasst.

11. Das digitale chirurgische Mikroskopiesystem nach Anspruch 1, wobei die mindestens eine Kamera zwei Kameras umfasst, die zur Aufnahme eines stereoskopischen Bildes ausgelegt sind.

12. Digitales chirurgisches Mikroskopiesystem nach Anspruch 1, das ferner eine Vielzahl von HMDs für jeden Benutzer umfasst, wodurch jeder Benutzer seinen jeweiligen ROI auswählen kann.

## Revendications

1. Système de microscopie chirurgicale numérique comprenant :
au moins une caméra, configurée pour acquérir des images d'une zone opératoire d'un patient ;
un casque de réalité virtuelle (HMD), configuré pour afficher à un utilisateur au moins une région d'intérêt (ROI) dans lesdites images ;
un dispositif de suivi configuré pour suivre les mouvements de la tête dudit utilisateur ; et
un dispositif de traitement configuré pour être couplé audit casque de visualisation, à ladite au moins une caméra et audit dispositif de suivi, ledit dispositif de traitement étant configuré pour commander au moins une fonction dudit système de microscopie chirurgicale numérique en fonction desdits mouvements,
dans lequel ladite au moins une fonction comprend au moins l'une des opérations suivantes : modifier un zoom optique ou numérique, modifier la taille de ladite zone d'intérêt, modifier l'emplacement de ladite zone d'intérêt, et commander les moteurs du microscope ;
dans lequel ledit système de microscopie chirurgicale numérique est un système non endoscopique.

2. Système de microscopie chirurgicale numérique selon la revendication 1, dans lequel ladite au moins une fonction comprend en outre la modification d'une image affichée via ledit casque de réalité virtuelle.

3. Système de microscopie chirurgicale numérique selon la revendication 2, dans lequel ladite image affichée via le casque de réalité virtuelle (HMD) comprend l'un des éléments suivants : ladite zone d'intérêt (ROI), les données relatives aux antécédents médicaux du patient, un scan du patient acquis au préalable, les données relatives aux signes vitaux du patient, et les données relatives à l'équipement de la salle d'opération.

4. Système de microscopie chirurgicale numérique selon la revendication 1, dans lequel lesdits moteurs du microscope comprennent au moins l'un parmi un moteur de zoom optique et un moteur XY.

5. Système de microscopie chirurgicale numérique selon la revendication 1, dans lequel ladite au moins une fonction comprend en outre au moins l'une parmi le contrôle de la transparence dudit casque de réalité virtuelle et la modification de la luminosité dudit casque de réalité virtuelle.

6. Système de microscopie chirurgicale numérique selon la revendication 1, dans lequel ledit système de microscopie chirurgicale numérique utilise l'activation vocale pour permettre ledit contrôle de ladite au moins une fonction.

7. Système de microscopie chirurgicale numérique selon la revendication 1, comprenant en outre des sources lumineuses configurées pour éclairer ladite zone opératoire.

8. Système de microscopie chirurgicale numérique selon la revendication 1, dans lequel ladite au moins une caméra est couplée à un bras robotisé, ladite au moins une fonction comprenant en outre le contrôle du mouvement dudit bras robotisé.

9. Système de microscopie chirurgicale numérique selon la revendication 1, dans lequel ledit dispositif de traitement est configuré pour effectuer un traitement d'image sur lesdites images acquises par ladite au moins une caméra.

10. Système de microscopie chirurgicale numérique selon la revendication 9, dans lequel ledit traitement d'images comprend en outre des corrections de bruit à motif fixe, des corrections de couleur, des corrections de distorsion, l'amélioration de l'histogramme, le suivi d'objets, la mise en évidence d'objets, la superposition de données de surveillance, la superposition de données d'antécédents du patient et la fusion avec d'autres sources d'images.

11. Système de microscopie chirurgicale numérique selon la revendication 1, dans lequel ladite au moins une caméra comprend deux caméras configurées pour capturer une image stéréoscopique.

12. Système de microscopie chirurgicale numérique selon la revendication 1, comprenant en outre une pluralité de casques de réalité virtuelle (HMD) pour chaque utilisateur, permettant à chaque utilisateur de sélectionner sa zone d'intérêt (ROI) respective.
